(19) Europäisches Patentamt
European Patent Office
Office européen des brevets

(11) EP 0 723 778 B1

(12) **FASCICULE DE BREVET EUROPEEN**

(45) Date de publication et mention de la délivrance du brevet:
**05.04.2000 Bulletin 2000/14**

(51) Int Cl.[7]: **A61K 31/11**, A61K 31/44

(21) Numéro de dépôt: **95402917.9**

(22) Date de dépôt: **22.12.1995**

(54) **Compostion induisant d'apoptose comprenant un facteur augmentant le taux intracellulaire de méthional ou de malondialdéhyde**

Zusammensetzung, die die Apoptose moduliert und einen Faktor enthält, der den intrazellulären Gehalt an Methional oder Malondialdehyd beeinflusst

Composition for induction of apoptosis comprising a factor increasing the intracellular level of methional or malondialdehyde

(84) Etats contractants désignés:
**AT BE CH DE DK ES FR GB GR IE IT LI LU MC NL PT SE**

(30) Priorité: **29.12.1994 FR 9415884**

(43) Date de publication de la demande:
**31.07.1996 Bulletin 1996/31**

(73) Titulaire: **CENTRE INTERNATIONAL DE RECHERCHES DERMATOLOGIQUES GALDERMA (C.I.R.D. GALDERMA)**
**F-06560 Valbonne (FR)**

(72) Inventeurs:
- **Quash, Georges Anthony**
  **F-69340 Francheville (FR)**
- **Doutheau, Alain**
  **F-69006 Lyon (FR)**

(74) Mandataire: **Tezier Herman, Béatrice**
**L'OREAL-DPI**
**6 rue Bertrand Sincholle**
**92585 Clichy Cédex (FR)**

(56) Documents cités:
**FR-A- 2 485 926** **FR-A- 2 612 396**
**FR-A- 2 673 530**

- **BIOCHEM. J., vol. 305, no. pt.3, 1995, pages 1017-1025, XP002003502 G. QUASH ET AL.: "Methional derived from 4-methylthio-2-oxobutanoate is a cellular mediator of apoptosis in baf3 lymphoid cells."**
- **BIOCHEM. PHARMACOL., vol. 45, no. 8, 1993, pages 1631-1644, XP002003503 G. OGIER ET AL.: "Contribution of 4-methylthio-2-oxobutanoate and its transaminase to the growth of methionine-dependent cells in culture: effect of transaminase inhibitors."**
- **CHEM. BIOL. INTERACT., vol. 50, no. 1, 1984, pages 87-96, XP002003504 F.W. SUMMERFIELD ET AL.: "Cross-linking of DNA in liver and testes of rats fed 1,3-propanediol."**
- **J.NEUROBIOL., vol. 24, no. 4, 1993, pages 433-446, XP002003505 M.D. LINNIK ET AL.: "Induction of programmed cell death in a dorsal root ganglia X neuroblastoma cell line."**
- **J. NEUROSCI., vol. 14, no. 7, 1994, pages 4385-4392, XP002003506 R.R. RATAN ET AL.: "Macromolecular synthesis inhibitors prevent oxidative stress-induced apoptosis in embryonic cortical neurons by shunting cysteine from protein synthesis to glutathione."**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne un composé choisi parmi le méthional, le malondialdéhyde et tout facteur augmentant le taux intracellulaire du méthional ou du malondialdéhyde pour une utilisation comme médicament. Ce médicament est plus particulièrement destiné à moduler le phénomène de la mort programmée des cellules (l'apoptose).

Elle concerne également une composition modulant l'apoptose. Elle a enfin pour objet un procédé pour prévenir et/ou lutter contre le vieillissement photoinduit ou chronologique de la peau.

Il existe deux types de mécanisme impliqués dans la mort des cellules. Le premier de type classique est appelé la nécrose. Morphologiquement, la nécrose est caractérisée par un gonflement des mitochondries et du cytoplasme et par une altération nucléaire, suivi de la destruction de la cellule et son autolyse, ceci étant accompagné par un phénomène d'inflammation. La nécrose survient de manière passive et incidente. La nécrose tissulaire est généralement due à un trauma physique des cellules ou un poison chimique, par exemple.

L'autre forme de mort cellulaire est appelée l'apoptose [Kerr, J.F.R. and Wyllie, A.H., Br. J. Cancer, 265, 239 (1972)], mais contrairement à la nécrose l'apoptose n'entraîne pas un phénomène d'inflammation. Il est décrit que l'apoptose peut se réaliser sous différentes conditions physiologiques. C'est une forme hautement sélective du suicide cellulaire qui se caractérise par des phénomènes morphologiques et biochimiques aisément observables. Ainsi, on observe notamment une condensation de la chromatine associée ou non à une activité endonucléasique, la formation de corps apoptiques et une fragmentation de l'acide désoxyribonucléique (A.D.N.) par l'activation d'endonucléases en des fragments d'A.D.N. de 180-200 paires de bases (ces fragments peuvent être observés par électrophorèse sur gel d'agarose).

L'apoptose peut être considérée comme une mort programmée des cellules impliquée dans le développement, la différenciation et l'homéostasie tissulaire. Il est donc considéré que la différenciation, la croissance et la maturation des cellules sont étroitement liées à l'apoptose. Ainsi, chez un être humain en bonne santé, il existe un équilibre entre l'ensemble de ces phénomènes.

Dans le domaine médical, un certain nombre de situations pathologiques présente un mécanisme d'apoptose modifié, voire déréglé, ou un mécanisme d'apoptose qui ne pourvoit pas à un dérèglement d'un autre phénomène biologique pour arriver à l'équilibre. Ainsi, il est décrit qu'une modulation volontaire de l'apoptose en l'induisant ou en la réprimant peut permettre de traiter de nombreuses maladies, plus particulièrement les maladies liées à une hyperprolifération cellulaire, telles que dans le cas du cancer, des maladies auto-immunes ou des allergies, ou au contraire les maladies liées à une disparition cellulaire, telle que dans les cas du syndrome d'immunodéficience du virus d'immunodéficience humain (H.I.V.), des maladies neuro-dégénératives (maladie d'Alzheimer), des dommages excessifs induits lors de l'infarctus du myocarde ou d'une lésion ischémique cérébrale.

De manière concrète, il a été constaté en oncologie que de nombreuses drogues anticancéreuses, telles que l'adriamycine et la cyclophosphamide, sont capables d'induire l'apoptose.

Dans le domaine de la cosmétique, les signes du vieillissement cutané résultent essentiellement d'un dysfonctionnement des principaux mécanismes biologiques de la peau faisant notamment intervenir le mécanisme de l'apoptose. On peut donc penser que tout produit venant moduler le mécanisme de l'apoptose est un produit apte à prévenir et/ou lutter contre l'apparition du vieillissement et les signes de vieillissement existants, comme les rides et les ridules.

Cependant, le lien entre les produits exogènes ou endogènes à la cellule et leur réponse cellulaire induisant ou réprimant l'apoptose n'est pas connu.

La Demanderesse a découvert que l'apoptose peut être induite par une augmentation du taux intracellulaire d'un métabolite naturel, le méthional (3-méthylthio-propanal) ou le malondialdéhyde. La Demanderesse a également découvert que la transformation in vivo du méthional en malondialdéhyde peut être dérégulée par l'expression d'oncogènes, tels que le gène $bcl_2$. Ce gène $bcl_2$ est décrit notamment comme étant capable d'inhiber l'apoptose induite par les espèces oxygénées réactives dans des cellules et plus particulièrement dans les cellules neuronales (Kane, D. J. et al., 1993, Science 262, 1274-1277).

Ainsi, la présente invention concerne un composé choisi parmi le méthional, le malondialdéhyde et tout facteur augmentant le taux intracellulaire du méthional ou du malondialdéhyde pour une utilisation comme médicament. Ce médicament est plus particulièrement destiné à moduler le phénomène de la mort programmée des cellules.

La présente invention a également pour objet une composition pharmaceutique ou cosmétique modulant l'apoptose, caractérisée en ce qu'elle comprend, à titre d'agent actif, un composé choisi parmi le méthional, le malondialdéhyde et tout facteur augmentant le taux intracellulaire du méthional ou du malondialdéhyde, en combinaison avec un support pharmaceutiquement ou cosmétiquement acceptable.

Le méthional, le malondialdéhyde et tout facteur augmentant le taux intracellulaire du méthional peuvent être utilisés dans la présente invention seuls ou de préférence en mélange. Bien entendu, le choix des produits est fait en fonction du but recherché, c'est à dire induire l'apoptose.

Ainsi, selon un mode particulier de réalisation de l'invention, la composition induisant l'apoptose, est caractérisée en ce qu'elle comprend, à titre d'agent actif, un composé choisi parmi le méthional, le malondialdéhyde et tout facteur augmentant le taux intracellulaire du méthional ou le malondialdéhyde, en combinaison avec un support pharmaceutiquement ou cosmétiquement acceptable.

Dans le métabolisme du méthional, il est connu que l'acide 4-méthylthio-2-oxobutanoïque peut être métabolisé in vivo par le complexe déshydrogénase oxo-acide à chaîne branchée présent dans les mitochondries des cellules du foie, du coeur, et du muscle squelettique via le méthional pour donner le méthylthiopropionylCoA [cf. Wu, G. & Yeaman, S.J. (1989) Biochem. J. 257, 281-284; Haussinger, D., Stehle, T. & Gerok, W. (1985) J. Biol. Chem. 366, 527-536; Jones, S.M.A. & Yeaman, S.J. (1986) Biochem. J. 237, 621-623]. Il est également décrit que l'acide 4-méthylthio-2-oxobutanoïque peut être métabolisé in vivo par transamination en méthionine [cf. Ogier, G., Chantepie, J., Deshayes, C., Chantegrel, B., Charlot, C., Doutheau, A & Quash, G. (1993) Biochem. Pharmacol. 45, 1631-1644]. Le méthional peut éventuellement aussi être réduit ou oxydé respectivement en méthionol par une aldéhyde réductase ou en acide méthylthio-propionique par une aldéhyde déshydrogénase. Le méthional en association avec le radical $HO^{\bullet}$ peut enfin donner du malondialdéhyde et du méthane thiol par une réaction de β-hydroxylation. Ainsi, afin de mieux situer le méthional, le malondialdéhyde et les facteurs pouvant influencer leur taux intracellulaire, la figure 1 illustre le métabolisme du méthional, sans toutefois limiter la portée de l'invention.

Dans cette figure 1 :

- MTOB représente l'acide 4-méthylthio-2-oxobutanoïque ;
- MTPA représente l'acide méthylthiopropionique ;
- E1 représente la décarboxylase du complexe déshydrogénase oxo-acide à chaîne branchée dont le co-facteur est la thiamine pyrophosphate (TPP) ;
- E2 représente la transacylase du complexe déshydrogénase oxo-acide à chaîne branchée dont le co-facteur est l'acide thioctique (TA) ;
- ALDR représente l'aldéhyde réductase ;
- ALDH représente l'aldéhyde déshydrogénase.

Par facteur augmentant le taux intracellulaire du méthional ou du malondialdéhyde, on entend selon l'invention les composés choisis parmi les précurseurs, les produits du méthional ou du malondialdéhyde, les inhibiteurs et les activateurs des enzymes impliquées dans le métabolisme du méthional ou du malondialdéhyde. Ainsi, on comprend aisément au vu du métabolisme du méthional ou du malondialdéhyde illustré à la figure 1 comment ces composés, le méthional ou le malondialdéhyde, introduits dans un système cellulaire, vont modifier le taux intracellulaire du méthional ou du malondialdéhyde de manière provisoire ou durable.

Les précurseurs ou les produits du méthional ou du malondialdéhyde peuvent être des précurseurs ou des produits du métabolisme intracellulaire du méthional, tels que l'acide 4-méthylthio-2-oxobutanoïque, la méthionine, le méthionol, l'acide méthylthiopropionique et le méthylthiopropionylCoA.

Les précurseurs ou les produits du méthional ou du malondialdéhyde peuvent être également des produits qui in situ ont la capacité de libérer du méthional ou du malondialdéhyde ou de libérer un facteur augmentant le taux intracellulaire de méthional ou du malondialdéhyde, tels que des esters et thioesters de méthional ou du malondialdéhyde qui vont libérer in situ du méthional, du malondialdéhyde ou l'acide 4-méthylthio-2-hydroxybutanoïque qui va être métabolisé in situ en acide 4-méthylthio-2-oxobutanoïque. Dans le domaine pharmaceutique, ces produits sont communément appelés des "prodrogues".

Dans le cas où l'on veut induire l'apoptose, le méthional, le malondialdéhyde, les précurseurs ou les produits du méthional ou du malondialdéhyde sont avantageusement utilisés en association avec des inhibiteurs d'enzymes impliquées dans des réactions métaboliques favorisant l'élimination de méthional autres que la réaction de β-hydroxylation mentionnée ci-dessus (qui transforme le méthional en malondialdéhyde) ou en association avec des activateurs de la β-hydroxylase qui est l'enzyme impliquée dans la transformation du méthional en malondialdéhyde. Ainsi, on peut citer, comme exemple, une association de l'acide 4-méthylthio-2-oxobutanoïque avec un inhibiteur de la transaminase impliquée dans la transformation de l'acide 4-méthylthio-2-oxobutanoïque en méthionine, tel que les inhibiteurs indiqués ci-dessous.

On peut également envisager l'association de méthional ou de facteur augmentant le taux intracellulaire du méthional avec des composés augmentant le taux de radical libre $HO^{\bullet}$. On peut ainsi citer le BCNU (N,N-Bis(2-chloroéthyl)-N-nitrosourea) car il est un inhibiteur de la glutathion réductase, ce qui permet d'augmenter le taux intracellulaire d'espèces oxygénées réactives, telles que le radical $HO^{\bullet}$ , qui est un des substrats de la réaction de β-hydroxylation pour donner du malondialdéhyde.

Cette association est particulièrement intéressante dans le cas de pathologies qui sont caractérisées par une surexpression du gène bcl2. De telles pathologies sont notamment les cancers du sein, les lymphomes des cellules B, les leucémies, les neuroblastomes, les adenocarcinomes de la prostate, les prolactinomas et autres adénomes pituitaires. La surexpression du gène bcl2 confère aux cellules un caractère chimiorésistant. Cette association peut

ainsi permettre d'inhiber partiellement, voire totalement, ce caractère chimiorésistant.

De manière avantageuse, on peut ajouter à cette association au moins un inhibiteur de la transaminase impliquée dans la transformation de l'acide 4-méthylthio-2-oxobutanoïque en méthionine, tels que ceux décrits ci-dessous et plus particulièrement le composé de formule (9).

Parmi les inhibiteurs ou les activateurs des enzymes impliquées dans le métabolisme du méthional, on peut notamment citer les inhibiteurs ou activateurs du complexe déshydrogénase oxo-acide à chaîne branchée impliqué dans la tranformation de l'acide 4-méthylthio-2-oxobutanoïque en méthylthiopropionylCoA via le méthional ou les inhibiteurs ou activateurs de là transaminase impliquée dans la transformation de l'acide 4-méthylthio-2-oxobutanoïque en méthionine ou les inhibiteurs ou activateurs de l'aldéhyde réductase responsable de la réduction de méthional en méthionol ou de l'aldéhyde déshydrogénase responsable de l'oxydation de méthional en acide méthylthio-propionique ou encore des activateurs ou inhibiteurs de la β-hydroxylase qui est l'enzyme impliquée dans la transformation du méthional en malondialdéhyde.

Ainsi, on peut citer, à titre d'inhibiteur du complexe déshydrogénase oxo-acide à chaîne branchée et donc à titre de facteur diminuant le taux intracellulaire du méthional ou du malondialdéhyde, le 2-oxobutyrate qui est un substrat bien connu de ce complexe [cf. Jones, S.M.A. & Cederbaum, A.I. (1980) Arch. of Biochem. and Biophys., 199, 438-447], ainsi que la cétoleucine, la cétoisoleucine et la cétovaline, qui sont d'autres substrats de ce complexe.

On peut citer également, à titre d'inhibiteurs de la transaminase impliquée dans la transformation de l'acide 4-méthylthio-2-oxobutanoïque en méthionine et donc à titre de facteurs augmentant le taux intracellulaire du méthional ou du malondialdéhyde, les produits décrits dans Ogier, G., Chantepie, J., Deshayes, C., Chantegrel, B., Charlot, C., Doutheau, A & Quash, G. (1993) Biochem. Pharmacol. 45, 1631-1644.

Plus particulièrement, les inhibiteurs de la transaminase impliquée dans la transformation de l'acide 4-méthylthio-2-oxobutanoïque en méthionine sont choisis parmi les produits de formules (1) à (9) suivantes :

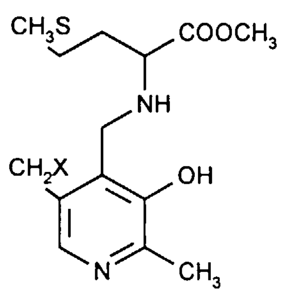

(1)

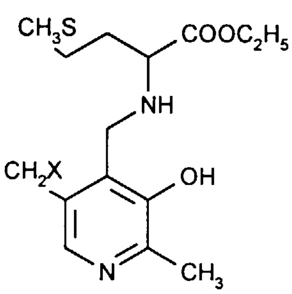

(2)

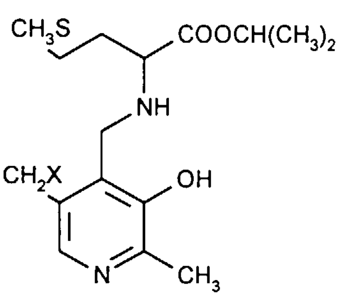

(3)

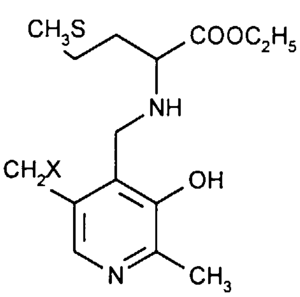

(4)

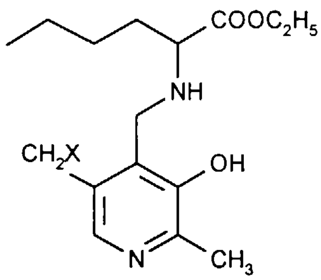

(5)

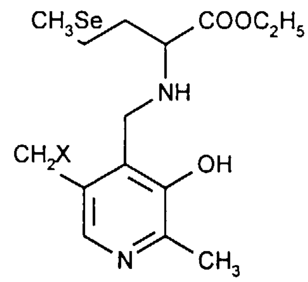

(6)

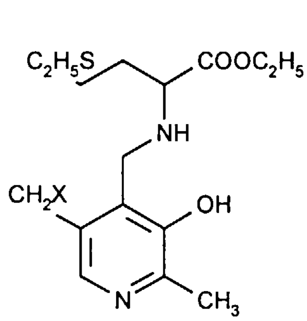

(7)

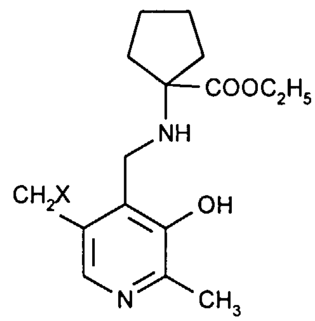

(8)

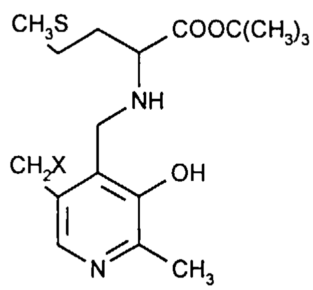

(9)

formules dans lesquelles X représente un radical -OH ou un radical $-OPO_3H_2$.

Le composé de formule (9) lorsque X représente un radical -OH est particulièrement préféré.

On peut encore citer, à titre d'inhibiteurs de la transaminase impliquée dans la transformation de l'acide 4-méthylthio-2-oxobutanoïque en méthionine et donc à titre de facteurs augmentant le taux intracellulaire du méthional ou du malondialdéhyde, les hydroxamates d'acides aminés amidés, tels que l'hydroxamate de la D-asparagine, de formule $CONHOHCH_2CHNH_2COOH$, et l'hydroxamate de la L-glutamine, de formule $CONHOHCH_2CH_2CHNH_2COOH$.

Lorsque l'on veut induire l'apoptose, on préfère selon l'invention utiliser un composé choisi parmi le méthional, le malondialdéhyde, des produits qui in situ ont la capacité de libérer du méthional ou du malondialdéhyde, tels que des esters ou thioesters de méthional ou de malondialdéhyde ou des activateurs de l'enzyme impliquée dans la transformation du méthional en malondialdéhyde.

Parmi les mélanges particulièrement intéressants pour induire l'apoptose, on peut particulièrement citer l'association de méthional avec au moins un inhibiteur de la transaminase impliquée dans la transformation de l'acide 4-méthylthio-2-oxobutanoïque en méthionine et préférentiellement le composé de formule (9).

Lorsque l'on veut réprimer l'apoptose, on préfère selon l'invention utiliser des inhibiteurs d'enzymes impliquées dans la production de méthional ou de malondialdéhyde, tel que le 2-oxobutyrate, et/ou des activateurs d'enzymes impliquées dans l'élimination du méthional autre que par la réaction de β-hydroxylation mentionnée ci-dessus.

La composition selon l'invention est plus particulièrement destinée aux traitements suivants:

1) Pour traiter les affections dermatologiques liées à un désordre de la kératinisation portant sur la différenciation et sur la hyperprolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.

2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).

3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale;

4) Pour traiter toutes les hyperproliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les hyperproliférations pouvant être induites par les ultra-violets notamment dans le cas des épithélioma baso et spinocellulaires.

5) Pour traiter d'autres désordres dermatologiques tels que les dermatoses bulleuses et les maladies du collagène.

6) Pour traiter certains troubles ophtalmologiques, notamment les cornéopathies.

7) Pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique.

8) Pour prévenir ou guérir les stigmates de l'atrophie épidermique et/ou dermique induite par les corticostéroïdes locaux ou systémiques, ou tout autre forme d'atrophie cutanée.

9) Pour prévenir ou traiter les troubles de la cicatrisation ou pour prévenir ou réparer les vergetures.

10) Pour lutter contre les troubles de la fonction sébacée tels que l'hyperséborrhée de l'acné ou la séborrhée simple.

11) Dans le traitement ou la prévention des états cancéreux ou précancéreux.

12) Dans le traitement d'affections inflammatoires telles que l'arthrite.

13) Dans le traitement de toute affection d'origine virale au niveau cutané ou général, telle que les hépatites.

14) Dans la prévention ou le traitement de l'alopécie.

15) Dans le traitement d'affections dermatologiques ou générales à composante immunologique.

16) Dans le traitement d'affections du système cardiovasculaire telles que l'artériosclérose et la thrombocytopénie.

17) Dans le traitement des maladies neuro-dégénératives, telles que la maladie d'Alzheimer.

Dans les domaines thérapeutiques mentionnés ci-dessus, la composition selon l'invention peut avantageusement comprendre d'autres agents actifs, tels que des rétinoïdes, des antiradicaux libres, des dérivés de la vitamine D, des corticostéroïdes ou des estrogènes, des anti-oxydants, des α-hydroxy ou α-céto acides ou leurs dérivés, ou encore des bloqueurs de canaux potassiques.

La présente invention a également pour objet l'utilisation de la composition selon l'invention dans ou pour la fabrication d'une composition cosmétique ou pharmaceutique destinée à moduler l'apoptose et plus particulièrement destinée aux traitements préventifs et/ou curatifs précédemment décrits.

Les rétinoïdes peuvent être d'origine naturelle ou synthétique. Parmi les rétinoïdes, on peut citer l'acide rétinoïque 9-*cis,* l'acide rétinoïque tout-*trans.*

Parmi les vitamines D ou leurs dérivés, on peut notamment citer les dérivés de la vitamine $D_2$ ou $D_3$ et en

particulier la 1,25-dihydroxyvitamine $D_3$.

Parmi les antiradicaux libres, on peut notamment citer l'α-tocophérol, la Super Oxyde Dismutase, l'Ubiquinol ou certains chélatants de métaux.

Parmi les α-hydroxy ou α-céto acides ou leurs dérivés, on peut notamment citer la cétoleucine, la cétoisoleucine, la cétovaline, le 2-oxobutyrate, l'acide 4-méthylthio-2-oxobutanoïque, les acides lactique, malique, citrique, glycolique, mandélique, tartrique, glycérique, ascorbique ou dérivés d'acide salicylique ou leurs sels, amides ou esters.

Parmi les bloqueurs de canaux potassiques, on peut notamment citer le Minoxidil (2,4-diamino-6-pipéridino-pyrimidine-3-oxyde) et ses dérivés.

L'administration de la composition selon l'invention peut être effectuée par voie entérale, parentérale, topique ou oculaire. De préférence, la composition est conditionnée sous une forme convenant à une application par voie systémique (pour injection ou perfusion).

Par voie entérale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Le médicament selon l'invention est généralement administré à une dose journalière d'environ 0,001 mg/kg à 100 mg/kg en poids corporel en 1 à 3 prises.

Par voie topique, la composition selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter soit sous forme anhydre, soit sous forme aqueuse.

Par voie oculaire, ce sont principalement des collyres.

La composition pharmaceutique par voie topique ou oculaire comprend du méthional, du malondialdéhyde ou tout facteur influençant le taux intracellulaire de méthional ou de malondialdéhyde à une concentration de préférence comprise entre 0,001 et 5 % par rapport au poids total de la composition.

La composition, selon l'invention, trouve également une application dans le domaine cosmétique, en particulier dans l'hygiène corporelle et capillaire et notamment pour le traitement des peaux à tendance acnéique, pour la repousse des cheveux, l'anti-chute, dans ia protection contre les effets néfastes du soleil ou dans le traitement des peaux physiologiquement sèches, pour prévenir et/ou lutter contre le vieillissement photo-induit ou chronologique.

La présente invention a enfin pour objet un procédé pour prévenir et/ou lutter contre le vieillissement photoinduit ou chronologique de la peau, caractérisé en ce que l'on applique sur la peau une composition cosmétique induisant l'apoptose telle que décrite précédemment.

Dans le domaine cosmétique, la composition selon l'invention peut avantageusement comprendre des rétinoïdes, des vitamines D ou leurs dérivés, des corticostéroïdes, des antiradicaux libres, des α-hydroxy- ou α-céto-acides ou leurs dérivés, ou encore des bloqueurs de canaux ioniques. Ces différents produits employés dans la composition de la présente invention étant tels que définis ci-dessus.

La composition cosmétique selon l'invention peut se présenter notamment sous forme d'une crème, d'un lait, d'une lotion, d'un gel, de microsphères ou nanosphères ou vésicules lipidiques ou polymériques, d'un savon ou d'un shampooing.

La concentration en méthional, en malondialdéhyde ou en tout facteur augmentant le taux intracellulaire de méthional ou de malondialdéhyde dans la composition cosmétique est préférentiellement comprise entre 0,0001 et 3 % en poids de la composition.

La composition pharmaceutique ou cosmétique selon l'invention peut, en outre, comprendre des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le b-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

La composition selon l'invention peut également contenir des agents d'amélioration de la saveur, des agents

conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'α-tocophérol, le butylhydroxyanisole ou le butylhydroxytoluène.

On va maintenant donner des exemples, à titre d'illustration et sans aucun caractère limitatif.

## EXEMPLES

### Exemple 1

Effet des métabolites sur la croissance des cellules HeLa

Des cellules HeLa (0,5 x $10^6$ par boîte de Petri) sont cultivées dans 3ml d'un milieu essentiel minimum de Eagle contenant 10 % en poids de serum de veau foetal dialysé. Après 4 heures, sont ajoutés à chacune des trois boîtes du méthional (10µM-1mM), du méthionol (10µM-20mM), de l'acide méthylthio-propionique (MTPA) (1µM-20mM), de la L-méthionine (1µM-20mM) ou de l'acide 4-méthylthio-2-oxobutanoïque (MTOB) (1µM-20mM). Après trois jours à 37°C, les cellules sont lavées deux fois dans un milieu salin tamponné à pH 7,5 et phosphaté (milieu PBS) et sont récupérées dans le même type de tampon.

La croissance cellulaire est évaluée en mesurant la teneur en protéines (cf. méthode dans Lowry, O.H., Rosebrouh, N.J., Farr, A.L. & Randall, R.J. (1951) J. BIOL. CHEM. 193, 265-275) ou la teneur en ADN dans les lysats utilisant une solution de trihydrochlorure de bisbenzimide (Hoechst 33258) (cf. Jarvis, W.D., Kolesnick, R.N., Fornari, F.A., Traylor, R.S., Gewirtz, D.A. & Grant, S. (1994) Proc. Natl. Acad. Sci.. U.S.A. 91, 73-77), ou en déterminant l'activité de la lactique déshydrogénase utilisant le bromure 3-(4,5-diméthylthiazol-2-yl)-2,5diphényl-tetrazolium (MTT) (cf. Mosmann, T. (1983) J. of Immunology. Methods 65, 55-63).

Les résultats sont rassemblés dans le tableau 1. Chaque valeur donnée est la moyenne de 3 ou 4 expériences estimées par la teneur en protéïnes.

Tableau 1

| Produit | IC50 (mM) |
|---|---|
| L-méthionine | >20 |
| MTOB | 1,6 |
| méthional | 0,10 |
| méthionol | >20 |
| MTPA | 10 |

IC50 correspond à la concentration de produit nécessaire pour obtenir une inhibition de croissance cellulaire de 50%.

Les résultats évalués en mesurant la teneur en ADN ou en déterminant l'activité de la lactique déshydrogénase sont comparables à ceux obtenus dans ce tableau.

Ce tableau montre la différence de IC50 du méthional par rapport aux IC50 de ses produits ou précurseurs.

### Exemple 2

Des cellules HeLa (0,5 x $10^6$ par boîte de Petri) sont cultivées dans 3ml de milieu essentiel minimum de Eagle dépourvu de méthionine ($Met^-$) contenant 10 % en poids de serum de veau foetal dialysé. Après 4 heures, est ajouté du 2-oxobutyrate (10mM - 40mM) en présence de 2mM d'acide 4-méthylthio-2-oxobutanoïque (MTOB). Après trois jours à 37°C, les cellules sont lavées deux fois dans un milieu salin tamponné à pH 7,5 et phosphaté (milieu PBS) et sont récupérées dans le même type de tampon. La croissance cellulaire est mesurée de la même manière que dans l'exemple précédent.

Le 2-oxobutyrate est un substrat bien connu du complexe déshydrogénase oxo-acide à chaîne branchée (BCOADC) (Km 18µM). Le MTOB seul à 2mM induit une inhibition de croissance de 72%. Les résultats montrent que le 2-oxobutyrate a une capacité de lever cette inhibition de croissance. L'inhibition est, à partir de 10mM de 2-oxobutyrate, réduite de 2 à 3 fois. Ainsi, on constate que l'activité du BCOADC pour la transformation de la MTOB en méthional est déplacée vers un substrat différent, tel que le 2-oxobutyrate, ce qui décroît l'inhibition de croissance.

**Exemple 3**

Effet du méthional sur la fragmentation de l'ADN dans les celllules BAF3

Les cellules utilisées correspondent à une lignée cellulaire lymphocytaire de souris BAF3 qui requiert de l'interleukine 3 (IL3) pour croître et qui subit l'apoptose (plus de 80% des cellules) en l'absence d'IL3 en 16 heures [cf. Collins, M.K.L., Marvel, J., Malde, P. & Lopez-Rivas, A. (1992) J. Exp. Med. 176, 1043-1051].

3 x $10^6$ cellules BAF3 sont cultivées dans 6ml d'un milieu de culture contenant de l'IL3 dans des boîtes de Petri en présence de différentes concentrations (200-800µM) de méthional. ou de propanal, qui est un substrat de l'aldéhyde déshydrogénase. Après 8 heures de contact, les cellules sont lavées trois fois dans un milieu salin tamponné à pH 7,5 et phosphaté (milieu PBS). Les cellules sont lysées dans 2ml à 0,1% de Triton X-100, 20mM d'EDTA (acide éthylènediaminetétraacétique), 5mM Tris pH 8 et ensuite centrifugées à 30.000g à 4°C pendant 30 minutes. Les surnageants sont décantés et sujets aux analyses suivantes.

A titre de comparaison, les mêmes cultures sont réalisées dans un milieu soit avec uniquement de l'IL3 (+IL3), soit sans IL3 (-IL3).

- Une analyse qualitative des fragments d'ADN obtenus est réalisée suivant la méthode décrite notamment dans la publication de Jarvis, W.D., Kolesnick, R.N., Fornari, F.A., Traylor, R.S., Gawirtz, D.A. & Grant, S. (1994) Proc. Natl. Acad. Sci. USA 91, 73-77. Les surnageants sont traités avec la ribonucléase A (20µg/ml), 1 heure à 37°C et avec la protéinase K (100µg/ml). L'ADN est purifié par extraction au phénol et précipité avec l'éthanol.

Dans l'extrait final, les fragments d'ADN de bas poids moléculaires sont analysés par électrophorèse sur gel d'agarose 1%. Après électrophorèse (60V, 3heures), les gels sont colorés par du bromure d'éthydium.

Les résultats montrent clairement que l'apoptose est induite avec le méthional, le gel obtenu présente l'image d'une échelle de fragments d'ADN multiples de 180 à 200 paires de bases typiques d'une induction d'apoptose. Dans le cas du propanal, le gel ne présente pas cette caractéristique.

- Une analyse est réalisée par spectrofluorométrie pour mesurer la quantité de fragments d'ADN obtenus (< 3 kilobases). On ajoute à 2ml de surnageant 1ml de solution de trihydrochlorure de bisbenzimide (Hoechst 33258) (1µg/ml) dans 3 mM de chlorure de sodium, 1mMd4EDTA, 10mM Tris pH 8. On analyse les solutions obtenues par fluorométrie (λ d'excitation = 365nm, λ d'émission = 460nm). Les quantités d'ADN sont calculées relativement à de l'ADN hautement purifié (0,1 à 1µg dans 2ml de tampon de lyse) traité comme indiqué ci-dessus et exprimées en $10^{-9}$ g d'ADN récupéré à partir de 3x$10^6$ cellules.

On obtient les résultats rassemblés dans le tableau 2 suivant.

Tableau 2

| Exemples | $10^{-9}$ g d'ADN/3x$10^6$ cellules |
|---|---|
| + IL3 | 63 |
| +IL3 + Méthional | 127 |
| -IL3 | 150 |

Ainsi, l'augmentation de la quantité de fragments d'ADN trouvés dans les cellules traitées avec IL3 et méthional est similaire à celle trouvée quand les cellules sont placées dans un milieu dépourvu d'IL3.

**Exemple 4**

Effet de BCNU et d'un inhibiteur de la transaminase impliquée dans la transformation de l'acide 4-méthylthio-2-oxobutanoïque en méthionine sur la production d'espèces oxygénées réactives (H2O2 et HO•)

1,5.$10^5$ cellules BAF3 (lignée cellulaire telle que décrite ci-dessus), ensemencées dans 3ml d'un milieu de culture contenant de l'IL3 [milieu de Eagle modifié de Dulbecco contenant 6% de sérum de veau foetal et 5% de milieu conditionné par des cellules Wehi-3B utilisé comme source de IL3, milieu décrit dans Collins et al. (1992) J. Exp. Med. 176, 1043-1051], sont traitées avec 10 µM de 2',7'-dichlorofluoresceine-diacétate (appelé ultérieurement DCFH-DA et obtenu par Molecular Probes Inc.). Après 1 heure d'incubation à 37°C, les cellules sont lavées avec un tampon PBS. 10 minutes avant l'analyse par fluorescence du dichlorofluoresceine (DCF) par cytométrie en flux, est ajouté 3 µg/ml

de iodure de propidium. L'incubation est réalisée en présence de différentes concentrations de BCNU et en présence ou non du composé A à 50 µM (qui correspond au composé de formule (9) lorsque X représente un radical -OH). Cette méthode permet de donner le pourcentage de fluorescence obtenu par la réaction des espèces oxygénées réactives (H2O2 et HO•) avec la DCFH-DA. La cytométrie en flux est réalisée avec un cytomètre à flux FACScan (Becton Dickinson, San Jose, CA, USA).

Les résultats sont rassemblés dans le tableau 3 suivant.

Tableau 3

| concentration en BCNU (µg/ml) | concentration en composé A (µM) | Fluorescence/$10^6$ cellules (%) |
|---|---|---|
| 10 | 0 | 4 |
| 20 | 0 | 4 |
| 40 | 0 | 10 |
| 10 | 50 | 2,5 |
| 20 | 50 | 1,5 |
| 40 | 50 | 1,75 |

Ainsi, ces résultats montrent que le composé A qui permet d'augmenter le taux de méthional, réprime l'augmentation de HO• dûe à l'augmentation de la concentration en BCNU. Ainsi, il est vraisemblable qu'il y ait une réaction du méthional avec les radicaux HO• présents pour donner du malondialdéhyde, qui induit alors l'apoptose.

## Exemple 5

Effet de BCNU et d'un inhibiteur de la transaminase impliquée dans la transformation de l'acide 4-méthylthio-2-oxobutanoïque en méthionine sur l'apoptose

Les coupures de brins d'ADN sont marquées avec du dUTP biotinylé (détecté par l'avidine fluoresceïne) in situ dans les cellules perméabilisées et fixées en utilisant le test de la transférase déoxynucléotidyle ADN terminale (TdT) tel que décrit dans Gorczyca et al., (1993) Cancer Res. 53, 1945-1951.

1,5.$10^6$ cellules BAF3 (lignée cellulaire telle que décrite ci-dessus) sont ensemencées dans 3ml d'un milieu de culture contenant de l'IL3 (milieu de Eagle modifié de Dulbecco contenant 6% de sérum de veau foetal et 5% de milieu conditionné par des cellules Wehi-3B utilisé comme source de IL3). Les cellules sont incubées en présence de 50 µM du composé A, de 40 µg/ml de BCNU, du mélange de ces deux produits (50 µM du composé A + 40 µg/ml de BCNU) ou en absence de ces deux composés (contrôle). Après 24 heures d'incubation, les cellules sont lavées dans le tampon PBS et fixées dans du formaldéhyde puis dans l'éthanol et laissées à -20°C pendant 3 jours. Puis les cellules sont remises en suspension dans 50 µl d'une solution contenant 0,1 µM de cacodylate de sodium, pH 7,5, 1mM CoCl2, 0,1 mM dithiothreitol, 0,05 mg/ml BSA (Bovin Serum Albumine), 10 unités de TdT (du thymus de veau de Boerhringer), 0,5 nmol de biotine-16dUTP (du thymus de veau de Boerhringer), 0,5 nmol de dATP, 0,5 nmol de dCTP et 0,5 nmol de dGTP pendant 1 heure à 37°C. Après lavage au PBS, les cellules sont remises en suspension dans 100 µl de tampon 4xSSC (0,15 M NaCl, 0,015 M de citrate de sodium) contenant 2,5 µg/ml d'avidine-fluoresceine isothiocyanate (Sigma), 0,1 % (poids/vol.) de RNAase A, 0,1 % triton X100 et 5 % (poids/vol.) de lait écrémé pendant 30 minutes à température ambiante dans l'obscurité. Ces cellules sont lavées puis mises en suspension dans 1ml de PBS contenant 3µg/ml de iodure de propidium. La cytométrie en flux est réalisée avec un cytomètre à flux FACScan (Becton Dickinson, San Jose, CA, USA).

Les résultats sont rasssemblés dans le tableau 4 suivant.

Tableau 4

| test | % cellules apoptotiques |
|---|---|
| contrôle | 0,2 |
| Composé A | 0,3 |
| BCNU | 2,3 |
| Composé A + BCNU | 41,1 |

A ces doses, les produits seuls induisent faiblement l'apoptose, par contre leur mélange induit fortement l'apoptose (synergie). Ce résultat confirme les résultats du tableau 3 et les conclusions y afférentes.

**Exemple 6**

Détermination de la combinaison thérapeutique la plus efficace pour traiter des souris greffées avec des cellules de mélanome

A t = O jour, $10^5$ cellules de mélanomes murins B16 F1 sont injectées dans des souris B6D2F1 (de la société IFFA CREDO, France). A t = 1 jour et ceci de manière continue pendant 15 jours, on injecte une fois par jour à ces souris différents régimes thérapeutiques spécifiés dans le tableau 3 ci-dessous. Le contrôle correspond à des souris n'ayant pas suivi de régime thérapeutique.
Le tableau 5 rassemble les résultats qui correspondent à des moyennes de résultats sur 3 souris traitées.

Tableau 5

| Régime thérapeutique (mg/kg) | Temps de survie moyen (jours) |
|---|---|
| contrôle | 20,5 |
| BCNU (0,1mg/kg) | 24,5 |
| Composé A (25mg/kg) | 21,9 |
| Méthional (25mg/kg) | 27 |
| BCNU + Méthional | 26 |
| Composé A + Méthional | 27 |
| BCNU + Composé A | 40,5 |
| BCNU + Composé A + Méthional | 57 |

Pour les régimes à plusieurs composés, les doses de chacun des composés utilisés sont identiques à celles des régimes lorsque les composés sont utilisés seuls.
Le temps de survie est compté à partir de t = 0 jour indiqué ci-dessus et correspond à une moyenne des souris n'ayant pas survécu au delà de 60 jours.
BCNU correspond à la N,N-Bis(2-chloroéthyl)-N-nitrosourea.
Composé A correspond au composé de formule (9) lorsque X représente un radical -OH.
Ainsi, on s'aperçoit que les deux derniers régimes sont particulièrement efficaces puisqu'ils augmentent de manière importante le temps de survie de ces souris. Parmi ces deux régimes, le dernier régime (additionné de méthional) permet d'obtenir le tiers des souris qui survit plus de 60 jours.

**Exemple 7**

On procède comme dans l'exemple précédent, les régimes et le nombre de souris traitées variant. Le tableau 6 rassemble ces données et les résultats obtenus.

Tableau 6

| Régime thérapeutique (mg/kg) | Nbre. souris traitées | Temps de survie moyen (jours) | % de survivants à long terme |
|---|---|---|---|
| contrôle | 6 | 23,7 | 0 |
| BCNU (0,1mg/kg) | 6 | 32,5 | 0 |
| Composé A (25mg/kg) + Méthional (50mg/kg) | 10 | 33,1 | 10 |
| BCNU + Composé A + Méthional | 10 | 36 | 50 |

Le temps de survie est compté à partir de t = 0 jour indiqué ci-dessus et correspond à une moyenne des

souris n'ayant pas survécu au delà de 60 jours.
Pour le dernier régime, les doses correspondent à celles des composés utilisés seuls.
Le pourcentage de survivants à long terme correspond au pourcentage de souris ayant survécu au delà de 60 jours.
Ainsi, on s'aperçoit que les deux derniers régimes, et surtout le dernier, sont particulièrement efficaces.

**Exemple 8**

Effet du méthional sur l'induction d'apoptose dans les cellules BAF3-b0 et BAF3-bcl2

Les cellules utilisées correspondent à une lignée cellulaire lymphocytaire BAF3 telle que décrite ci-dessus. Les cellules BAF3-bcl2 correspondent à des cellules BAF3 transfectées par le gène bcl2, les cellules BAF3-b0 correspondent à des cellules BAF3 non transfectées par le gène bcl2. Comme précisé précédemment, les cellules BAF3-b0 subissent l'apoptose (plus de 80% des cellules) en l'absence d'IL3 en 16 heures. Par contre, les cellules BAF3-bcl2 qui sont donc transfectées par le gène bcl2 ne présentent aucun signe d'apoptose en l'absence d'IL3. De ce fait, elles constituent un bon modèle pour déterminer l'étape d'apoptose bloquée dans ces cellules et sa corrélation éventuelle avec l'inhibition de la synthèse du malondialdéhyde.
Les celllules BAF3-b0 ou BAF3-bcl2 sont marquées par une adaptation de la méthode décrite dans Wright, S. et al. (1992) J. of Cell. Biochem. 48, 344-355, en incubant $2,5.10^5$ cellules /ml avec 0,5 µCi [3H]-thymidine pendant 40 heures à 37°C. Après deux lavages avec un milieu de culture, $2,5.\ 10^6$ cellules sont mises en culture en présence de méthional. Après incubation pendant 8 heures, ces cellules sont récupérées par centrifugation à 400g pendant 5 minutes et lavées 3 fois dans du tampon PBS. Les cellules récupérées dans le culot sont lysées dans 2ml de 0,1% de Triton X-100, 20mM EDTA, 5mM Tris pH8 et centrifugées à 30000g à 4°C pendant 30 minutes. Les surnageants sont récupérés et les culots dissous dans 0,3 ml de 0,5 N NaOH. Des aliquots du milieu de culture (1ml), du surnageant (0,3ml) et du culot solubilisé (0,1ml) sont mis à doser dans le compteur à scintillation. Le pourcentage de fragments d'ADN est calculé de la façon suivante :

$$\text{\% de fragments d'ADN} = \frac{\text{dpm du milieu de culture + dpm du surnageant}}{\text{dpm du milieu de culture + dpm du surnageant + dpm du culot solubilisé}}$$

Résultats : l'addition d'une concentration croissante en méthional (0; 50; 100; 200; 300; 400 µM) dans le milieu d'incubation augmente le pourcentage de fragments d'ADN jusqu'à un maximum, pour 400 µM de méthional, de 26% comparé à 7% pour les cellules BAF3-bo non traitées par le méthional (contrôle). Pour les cellules BAF3-bcl2 traitées avec des concentrations équivalentes en méthional, les fragments d'ADN atteignent un maximum de 7%, pour 400 µM de méthional, comparé à 3% pour le contrôle.
Ces résultats montrent clairement que l'ajout du méthional ne permet pas de lever l'inhibition d'apoptose dûe au gène bcl2 dans les cellules BAF3-bcl2.
Lors de l'induction de l'apoptose par carence en IL3, il est observé que le méthional est transformé en malondialdéhyde par une réaction de β-hydroxylation à l'aide de $HO^\bullet$. Nous avons donc entrepris le dosage de H202 et de $HO^\bullet$ dans les cellules BAF3-b0 et BAF3-bcl2.

**Exemple 9**

Mesure des espèces oxygénées réactives (H202 et $OH^\bullet$) par la méthode de cytométrie en flux

Les cellules utilisées correspondent à une lignée cellulaire lymphocytaire BAF3 telle que décrite ci-dessus. Les cellules BAF3-bcl2 correspondent à des cellules BAF3 transfectées par le gène bcl2, les cellules BAF3-b0 correspondent à des cellules BAF3 non transfectées par le gène bcl2.
$1,5.10^6$ cellules BAF3-b0 ou -bcl2, ensemencées dans 3ml d'un milieu de culture contenant de l'IL3, sont traitées avec 10 µM de dichlorofluoresceine-diacétate (DCFH-DA). Après 1 heure d'incubation à 37°C, les cellules sont lavées avec un tampon PBS. 10 minutes avant l'analyse par fluorescence du dichlorofluoresceine (DCF) par cytométrie en flux, est ajouté 3 µg/ml de iodure de propidium, comme dans l'exemple 4. La cytométrie en flux est réalisée avec un cytomètre à flux FACScan (Becton Dickinson, San Jose, CA, USA).
Les résultats sont rassemblés dans le tableau 7 suivant.

Tableau 7

| type cellulaire | moyenne d'unité de fluorescence arbitraire |
|---|---|
| BAF3-b0 | 154 ± 32 |
| BAF3-bcl2 | 211 ± 52 |

Par conséquent, même s'il existe une tendance pour les cellules BAF3-bcl2 d'avoir plus d'espèces oxygénées réactives que les cellules BAF3-b0, cette différence n'est pas significative.

Il est donc possible que les cellules BAF3-bcl2 possèdent un défaut dans la synthèse de méthional et/ou de malondialdéhyde.

**Exemple 10**

Analyse des produits marqués au $^{14}C$ dérivés de [$^{14}C$]MTOB dans des cellules BAF3-b0 et BAF3-bcl2

Les cellules utilisées sont donc identiques à l'exemple précédent.

Le [$^{14}C$]MTOB est préparé par déamination oxydative du [$^{14}C$]Methionine tel que décrit dans Ogier G. et al. (1993) Biochem. Pharmacol. 45, 1631-1644. [$^{14}C$]MTOB ($5.10^6$ dpm correspondant à 38,2 nmol) est ajouté à une suspension cellulaire de BAF3-b0 ou BAF3-bcl2 ($2,4.10^5$ cellules/ml) dans du DMEM contenant 6% de sérum de veau foetal et 5% d'IL3. 39 heures plus tard, les cellules sont lavées deux fois dans du PBS et remises en suspension à $9.10^5$ cellules/ml. A $10^8$ cellules est ajouté [U $^{14}C$]MTOB ($1,9.10^6$ dpm correspondant à 14,5 nmol) et 8 heures plus tard les cellules sont récupérées par centrifugation à 400g pendant 5 minutes. Les culots celluleires sont solubilisés avec 1,5 ml de 0,5 N NaOH pendant 30 minutes à 60°C. La radioactivité totale présente dans les cellules est mesurée sur des aliquots de 10 µl. Après cela, est ajouté à l'hydrolysat alcalin obtenu de l'acide perchlorique à une concentration finale de 0,5 N, puis 200 µmol de 2,4-DNPH (2,4-dinitrophenylhyrazine, obtenu par Merck). Le mélange est chauffé à 70°C pendant 30 minutes, puis centrifugé à 400g pendant 5 minutes. Le précipité est solubilisé dans 1 ml de 0,5 N NaOH, la radioactivité et la teneur en protéines sont déterminées. Au surnageant est ajouté du NaOH à pH 10. Les métabolites marqués sont extraits dans de l'acétate d'éthylène et la phase organique séchée, acidifiée et extraite dans du dichlorométhane. Les métabolites marqués dans la phase organique sont séparés et identifiés par h.p.-tlc (highperformance-thin layer chromatography) et comparés aux dérivés du 2,4-DNPH des composés de référence puis quantifiés par radiochromatographie à balayage.

Les résultats sont rassemblés dans le tableau 8 suivant :

| | BAF3-b0 | BAF3-bcl2 |
|---|---|---|
| radio. (dpm/mg d'ADN) | 1 252 000 | 1 111 000 |
| activité spéc. de protéines cellulaires (dpm/mg prot.) | 23 500 | 20 860 |
| méthionine libre (dpm/mg d'ADN) | 36 820 | 51 440 |
| 2,4-DNPH méthional (dpm/mg d'ADN) | 14 050 | 7 650 |
| 2,4-DNPH MDA (2,4-DNPH pyrazole) (dpm/mg d'ADN) | 800 | 0 |

Tableau 8

Radio. signifie radioactivité totale présente dans les cellules.
MDA signifie malondialdéhyde.

Ainsi, il apparaît que la radioactivité totale rapportée à une quantité d'ADN identique est similaire dans les deux types cellulaires après 47 heures d'incubation. Il en est de même pour l'activité de la méthionine des protéines cellulaires. En ce qui concerne la méthionine libre, une augmentation de 40 % de la radioactivité est observée dans

les cellules BAF3-bcl2 comparée à celle dans les BAF3-b0. La radioactivité présente dans le méthional est 80% plus grande dans les cellules BAF3-b0 que dans les cellules BAF3-bcl2. Ceci permet de démontrer que dans les cellules BAF3-bcl2 la diminution de la formation de méthional à partir de MTOB est accompagnée d'une augmentation concommitante de la formation de méthionine. Quand la formation de MDA marqué est mesurée, celle-ci correspond à 800 dpm/mg d'ADN dans les cellules BAF3-b0, alors qu'aucune radioactivité pour le MDA n'est décelée dans les extraits des cellules BAF3-bcl2. Les cellules BAF3-bcl2 présentent donc également une diminution dans la formation de MDA à partir de méthional. L'exemple 8 montre clairement que cette diminution n'est pas dûe à une diminution des espèces oxygénées réactives dans les cellules BAF3-bcl2 comparativement aux cellules BAF3-b0. On peut donc conclure que le gène bcl2 en plus de diminuer la transformation du MTOB en méthional agit de manière négative aussi sur la réaction de β-hydroxylation transformant le méthional en malondialdéhyde. Donc, tout produit qui module le taux intracellulaire de méthional ou son produit de métabolisme, le malondialdéhyde, peut être considéré comme un modulateur de l'apoptose.

## Revendications

1. Composé choisi parmi le méthional, le malondialdéhyde et tout facteur augmentant le taux intracellulaire du méthional ou du malondialdéhyde pour une utilisation comme médicament.

2. Composition pharmaceutique ou cosmétique induisant l'apoptose, caractérisée en ce qu'elle comprend, à titre d'agent actif, au moins un composé choisi parmi le méthional, le malondialdéhyde et tout facteur augmentant le taux intracellulaire du méthional ou du malondialdéhyde, en combinaison avec un support pharmaceutiquement ou cosmétiquement acceptable.

3. Composition selon la revendication 2, caractérisée en ce que le méthional, le malondialdéhyde et tout facteur augmentant le taux intracellulaire du méthional ou du malondialdéhyde sont utilisés en mélange.

4. Composition selon l'une des revendications 2 ou 3, caractérisée en ce que les facteurs augmentant le taux intracellulaire du méthional ou du malondialdéhyde sont choisis parmi les précurseurs, les produits du méthional, les inhibiteurs et les activateurs des enzymes impliquées dans le métabolisme du méthional.

5. Composition selon la revendication précédente, caractérisée en ce que les précurseurs ou les produits du méthional ou du malondialdéhyde sont des précurseurs ou des produits du métabolisme intracellulaire du méthional ou du malondialdéhyde, tels que l'acide 4-méthylthio-2-oxobutanoïque, la méthionine, le méthionol, l'acide méthylthio-propionique et le méthylthiopropionylCoA.

6. Composition selon la revendication 4, caractérisée en ce que les précurseurs ou les produits du méthional ou du malondialdéhyde sont des produits qui in situ ont la capacité de libérer du méthional ou du malondialdéhyde ou de libérer un facteur augmentant le taux intracellulaire du méthional ou du malondialdéhyde, tels que des esters et thioesters de méthional ou de malondialdéhyde.

7. Composition selon la revendication 4, caractérisée en ce que les inhibiteurs ou les activateurs des enzymes impliquées dans le métabolisme du méthional sont choisis parmi les inhibiteurs ou activateurs du complexe déshydrogénase oxo-acide à chaîne branchée impliqué dans la tranformation de l'acide 4-méthylthio-2-oxobutanoïque en méthylthiopropionylCoA via le méthional, les inhibiteurs de la transaminase impliquée dans la transformation de l'acide 4-méthylthio-2-oxobutanoïque en méthionine, les inhibiteurs de l'aldéhyde réductase responsable de la réduction de méthional en méthionol ou de l'aldéhyde déshydrogénase responsable de l'oxydation de méthional en acide méthylthio-propionique et les inhibiteurs ou activateurs de la β-hydroxylase.

8. Composition selon la revendication 2, caractérisée en ce que les facteurs augmentant le taux intracellulaire du méthional ou du malondialdéhyde sont des inhibiteurs de la transaminase impliquée dans la transformation de l'acide 4-méthylthio-2-oxobutanoïque en méthionine choisis parmi les produits de formules (1) à (9) suivantes :

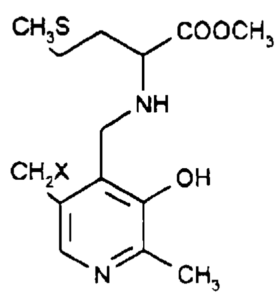

(1)

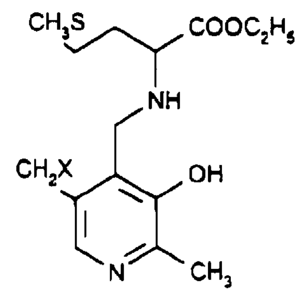

(2)

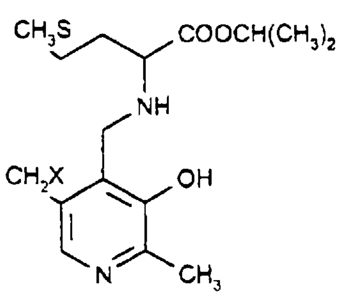

(3)

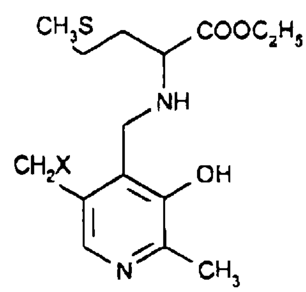

(4)

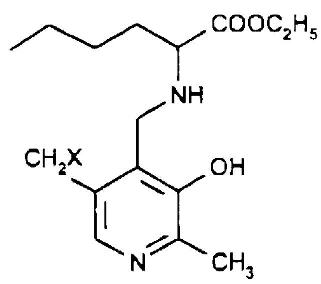

(5)

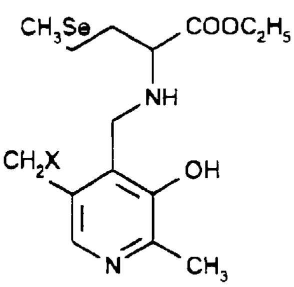

(6)

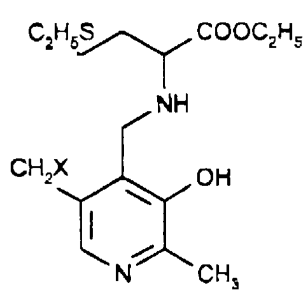

(7)

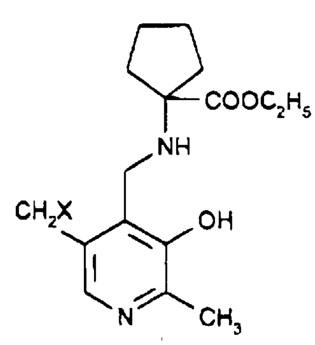

(8)

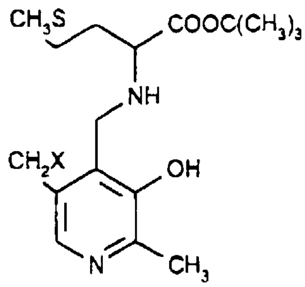

(9)

formules dans lesquelles X représente -OH ou $-OPO_3H_2$.

9. Composition selon la revendication 8, caractérisée en ce que l'inhibiteur de la transaminase impliquée dans la transformation de l'acide 4-méthylthio-2-oxobutanoïque en méthionine est le composé de formule (9) dans laquelle X représente le radical -OH.

10. Composition selon la revendication 2, caractérisée en ce que les facteurs augmentant le taux intracellulaire du méthional ou du malondialdéhyde sont des inhibiteurs de la transaminase impliquée dans la transformation de l'acide 4-méthylthio-2-oxobutanoïque en méthionine choisis parmi les hydroxamates d'acides aminés amidés.

11. Composition selon la revendication 2, caractérisée en ce qu'elle comprend une association d'un composé choisi parmi le méthional, le malondialdéhyde, les précurseurs et les produits du méthional ou du malondialdéhyde avec des inhibiteurs d'enzymes impliqués dans des réactions métaboliques favorisant l'élimination de méthional ou avec des activateurs de la β-hydroxylase.

12. Composition selon la revendication précédente, caractérisée en ce que l'association correspond à l'acide 4-méthylthio-2-oxobutanoïque avec un inhibiteur de la transaminase impliquée dans la transformation de l'acide 4-méthylthio-2-oxobutanoïque en méthionine, tel que les produits mentionnés dans l'une des revendications 8 à 10.

13. Composition selon la revendication 2, caractérisée en ce que l'association correspond au méthional ou à un facteur augmentant le taux intracellulaire du méthional avec au moins un composé augmentant le taux de radical libre $HO^{\bullet}$, ce composé étant de préférence le BCNU (N,N-Bis(2-chloroéthyl)-N-nitrosouréa).

14. Composition selon la revendication précédente, caractérisée en ce qu'elle comprend au moins un inhibiteur de la transaminase impliquée dans la transformation de l'acide 4-méthylthio-2-oxobutanoïque en méthionine, tel que les produits mentionnés dans l'une des revendications 8 à 10.

15. Utilisation d'une composition définie selon l'une quelconque des revendications 2 à 14 dans une composition cosmétique ou pour la fabrication d'une composition pharmaceutique, la composition étant destinée à induire l'apoptose.

16. Procédé autre qu'une méthode de traitement thérapeutique pour prévenir et/ou lutter contre le vieillissement photoinduit ou chronologique de la peau, caractérisé en ce que l'on applique sur la peau une composition cosmétique induisant l'apoptose selon l'une des revendications 2 à 14.

**Patentansprüche**

1. Verbindung, die unter Methional, Malondialdehyd und beliebigen Faktoren, die den intracellulären Gehalt an Methional oder Malondialdehyd erhöhen, ausgewählt ist, zur Verwendung als Arzneimittel.

2. Pharmazeutische oder kosmetische, die Apoptose induzierende Zusammensetzung, dadurch gekennzeichnet, daß sie als Wirkstoff mindestens eine Verbindung, die unter Methional, Malondialdehyd und beliebigen Faktoren, die den intracellulären Gehalt an Methional oder Malondialdehyd erhöhen, ausgewählt ist, in Kombination mit

einem pharmazeutisch oder kosmetisch akzeptablen Träger enthält.

**3.** Zusammensetzung nach Anspruch 2, dadurch gekennzeichnet, daß das Methional, der Malondialdehyd und die beliebigen Faktoren, die den intracellulären Gehalt an Methional oder Malondialdehyd erhöhen, im Gemisch verwendet werden.

**4.** Zusammensetzung nach einem der Ansprüche 2 oder 3, dadurch gekennzeichnet, daß die Faktoren, die den intracellulären Gehalt an Methional oder Malondialdehyd erhöhen, unter den Vorprodukten und den Produkten von Methional sowie den Inhibitoren und Aktivatoren der am Methional-Stoffwechsel beteiligten Enzyme ausgewählt sind.

**5.** Zusammensetzung nach dem vorhergehenden Anspruch, dadurch gekennzeichnet, daß die Vorprodukte oder die Produkte von Methional oder Malondialdehyd Vorprodukte oder Produkte des intracellulären Stoffwechsels von Methional oder Malondialdehyd sind, wie 4-Methylthio-2-oxobuttersäure, Methionin, Methionol, Methylthiopropionsäure und Methylthiopropionyl-CoA.

**6.** Zusammensetzung nach Anspruch 4, dadurch gekennzeichnet, daß die Vorprodukte oder die Produkte von Methional oder Malondialdehyd Produkte sind, die in situ die Fähigkeit aufweisen, Methional oder Malondialdehyd freizusetzen oder einen Faktor freizusetzen, der den intracellulären Gehalt an Methional oder Malondialdehyd erhöht, wie Ester und Thioester von Methional oder Malondialdehyd.

**7.** Zusammensetzung nach Anspruch 4, dadurch gekennzeichnet, daß die Inhibitoren oder die Aktivatoren der am Methional-Stoffwechsel beteiligten Enzyme ausgewählt sind unter den Inhibitoren oder Aktivatoren des Verzweigtketten-Oxosäure-Dehydrogenase-Komplexes, der an der Umwandlung der 4-Methylthio-2-oxobuttersäure in Methylthiopropionyl-CoA über Methional beteiligt ist, den Inhibitoren der Transaminase, die an der Umwandlung der 4-Methylthio-2-oxobuttersäure in Methionin beteiligt ist, den Inhibitoren der Aldehyd-Reduktase, die für die Reduktion von Methional zu Methionol verantwortlich ist, oder der Aldehyd-Dehydrogenase, die für die Oxidation von Methional zu Methylthiopropionsäure verantwortlich ist, und den Inhibitoren oder Aktivatoren der β-Hydroxylase.

**8.** Zusammensetzung nach Anspruch 2, dadurch gekennzeichnet, daß die Faktoren, die den intracellulären Gehalt an Methional oder Malondialdehyd erhöhen, Inhibitoren der an der Umwandlung der 4-Methylthio-2-oxobuttersäure in Methionin beteiligten Transaminase sind, die unter den Produkten der folgenden Formeln (1) bis (9) ausgewählt sind:

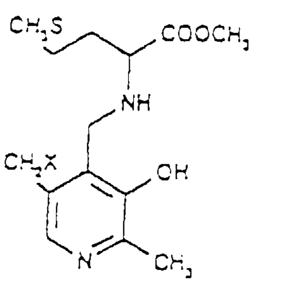

(1)

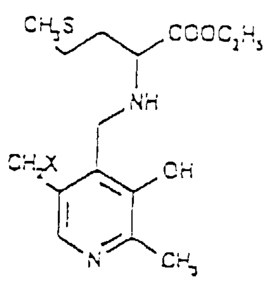

(2)

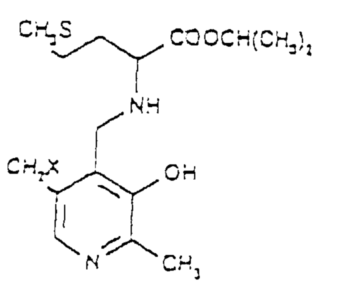

(3)

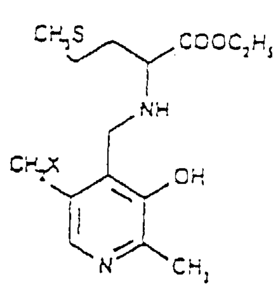

(4)

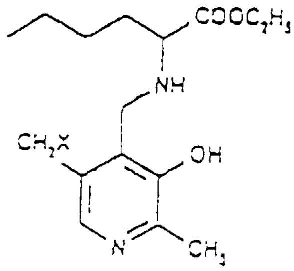

(5)

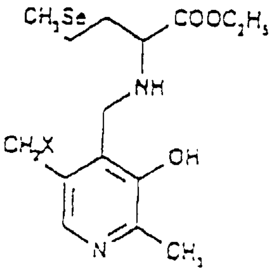

(6)

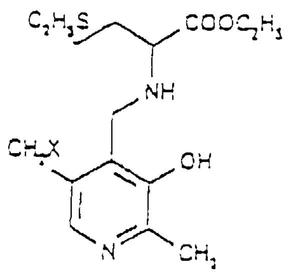

(7)

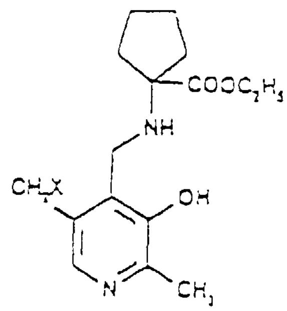

(8)

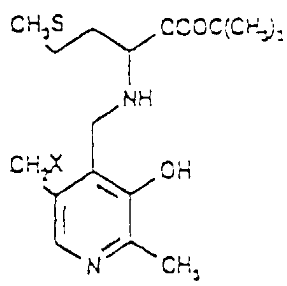

(9)

wobei in den Formeln X -OH oder $-OPO_3H_2$ bedeutet.

**9.** Zusammensetzung nach Anspruch 8, dadurch gekennzeichnet, daß der Inhibitor der Transaminase, die an der Umwandlung der 4-Methylthio-2-oxobuttersäure in Methionin beteiligt ist, die Verbindung der Formel (9) ist, worin X die Gruppe -OH bedeutet.

**10.** Zusammensetzung nach Anspruch 2, dadurch gekennzeichnet, daß die Faktoren, die den intracellulären Gehalt an Methional oder Malondialdehyd erhöhen, Inhibitoren der an der Umwandlung der 4-Methylthio-2-oxobuttersäure in Methionin beteiligten Transaminase sind, die unter den Hydroxamaten von amidhaltigen Aminosäuren ausgewählt sind.

**11.** Zusammensetzung nach Anspruch 2, dadurch gekennzeichnet, daß sie eine Kombination einer Verbindung, die unter Methional, Malondialdehyd, den Vorprodukten und den Produkten von Methional oder Malondialdehyd ausgewählt ist, mit Inhibitoren von Enzymen, die an den die Eliminierung von Methional fördernden Stoffwechselreaktionen beteiligt sind, oder mit Aktivatoren der β-Hydroxylase enthält.

**12.** Zusammensetzung nach dem vorhergehenden Anspruch, dadurch gekennzeichnet, daß die Kombination einer

Kombination von 4-Methylthio-2-oxobuttersäure mit einem Inhibitor der Transaminase, die an der Umwandlung der 4-Methylthio-2-oxobuttersäure in Methionin beteiligt ist, wie den in einem der Ansprüche 8 bis 10 genannten Produkten, entspricht.

**13.** Zusammensetzung nach Anspruch 2, dadurch gekennzeichnet, daß die Kombination einer Kombination von Methional oder einem Faktor, der den intracellulären Gehalt an Methional erhöht, mit mindestens einer Verbindung entspricht, die den Anteil des freien •OH-Radikals erhöht, wobei diese Verbindung vorzugsweise BCNH (N,N-Bis-(2-chlorethyl)-N-nitrosoharnstoff) ist.

**14.** Zusammensetzung nach dem vorhergehenden Anspruch, dadurch gekennzeichnet, daß sie mindestens einen Inhibitor der Transaminase, die an der Umwandlung der 4-Methylthio-2-oxobuttersäure in Methionin beteiligt ist, enthält, wie die in einem der Ansprüche 8 bis 10 genannten Produkte.

**15.** Verwendung einer nach einem der Ansprüche 2 bis 14 definierten Zusammensetzung in einer kosmetischen Zusammensetzung oder zur Herstellung einer pharmazeutischen Zusammensetzung, wobei die Zusammensetzung dazu bestimmt ist, die Apoptose zu induzieren.

**16.** Verfahren zur Vorbeugung und/oder Bekämpfung der lichtinduzierten oder altersbedingten Hautalterung, das von einer therapeutischen Behandlungsmethode verschieden ist, dadurch gekennzeichnet, daß eine die Apoptose induzierende kosmetische Zusammensetzung nach einem der Ansprüche 2 bis 14 auf die Haut aufgetragen wird.

### Claims

**1.** Compound chosen from methional, malondialdehyde and any factor which increases the intracellular level of methional or of malondialdehyde, for use as a medicinal product.

**2.** Pharmaceutical or cosmetic composition which induces apoptosis, characterized in that it comprises, as active agent, at least one compound chosen from methional, malondialdehyde and any factor which increases the intracellular level of methional or of malondialdehyde, in combination with a pharmaceutically or cosmetically acceptable support.

**3.** Composition according to Claim 2, characterized in that the methional, the malondialdehyde and any factor which increases the intracellular level of methional or of malondialdehyde are used as a mixture.

**4.** Composition according to either of Claims 2 and 3, characterized in that the factors increasing the intracellular level of methional or of malondialdehyde are chosen from the precursors or products of methional, and the inhibitors and activators of the enzymes involved in the metabolism of methional.

**5.** Composition according to the preceding claim, characterized in that the methional or malondialdehyde precursors or products are precursors or products of the intracellular metabolism of methional or of malondialdehyde, such as 4-methylthio-2-oxobutanoic acid, methionine, methionol, methylthiopropionic acid and methylthiopropionylCoA.

**6.** Composition according to Claim 4, characterized in that the methional or malondialdehyde precursors or products are products which, in situ, have the capacity to liberate methional or malondialdehyde or to liberate a factor which increases the intracellular level of methional or of malondialdehyde, such as esters and thioesters of methional or of malondialdehyde.

**7.** Composition according to Claim 4, characterized in that the inhibitors or activators of the enzymes involved in the metabolism of methional are chosen from the inhibitors or activators of the branched-chain oxo-acid dehydrogenase complex involved in the conversion of 4-methylthio-2-oxobutanoic acid into methylthiopropionylCoA via methional, the inhibitors of the transaminase involved in the conversion of 4-methylthio-2-oxobutanoic acid into methionine, the inhibitors of aldehyde reductase responsible for the reduction of methional to methionol or of aldehyde dehydrogenase responsible for the oxidation of methional to methylthiopropionic acid, and the activators or inhibitors of β-hydroxylase.

**8.** Composition according to Claim 2, characterized in that the factors which increase the intracellular level of methional or of malondialdehyde are inhibitors of the transaminase involved in the conversion of 4-methylthio-2-oxobutanoic acid into methionine which are chosen from the products of the following formulae (1) to (9):

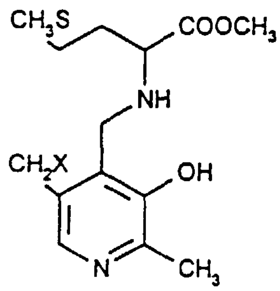

(1)

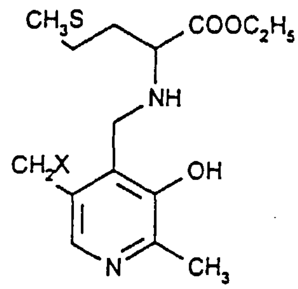

(2)

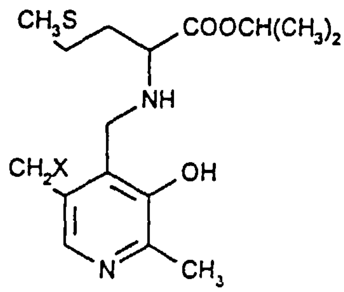

(3)

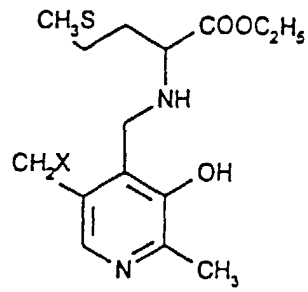

(4)

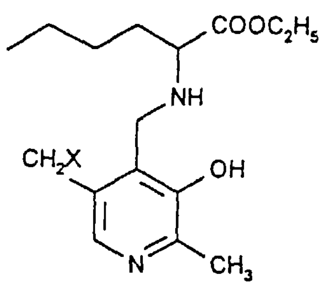

(5)

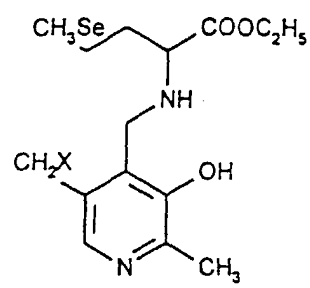

(6)

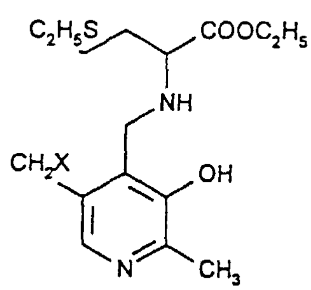

(7)

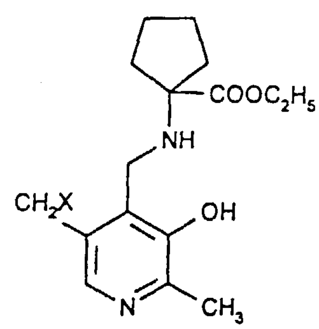

(8)

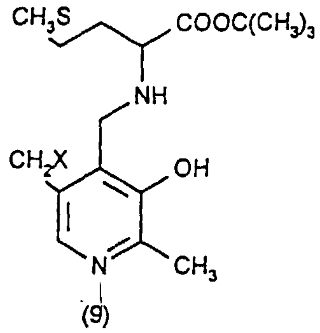

(9)

in which formulae X represents -OH or $-OPO_3H_2$.

**9.** Composition according to Claim 8, characterized in that the inhibitor of the transaminase involved in the conversion of 4-methylthio-2-oxobutanoic acid into methionine is the compound of formula (9) in which X represents the -OH radical.

**10.** Composition according to Claim 2, characterized in that the factors which increase the intracellular level of methional or of malondialdehyde are inhibitors of the transaminase involved in the conversion of 4-methylthio-2-oxobutanoic acid into methionine which are chosen from the hydroxamates of amido amino acids.

**11.** Composition according to Claim 2, characterized in that it comprises a combination of a compound chosen from methional, malondialdehyde, and the precursors and products of methional or of malondialdehyde, with inhibitors of enzymes involved in metabolic reactions promoting the removal of methional or with β-hydroxylase activators.

**12.** Composition according to the preceding claim, characterized in that the combination corresponds to 4-methylthio-2-oxobutanoic acid with an inhibitor of the transaminase involved in the conversion of 4-methylthio-2-oxobutanoic acid into methionine, such as the products mentioned in one of Claims 8 to 10.

**13.** Composition according to Claim 2, characterized in that the combination corresponds to methional or to a factor which increases the intracellular level of methional with at least one compound which increases the level of free radical $HO^\bullet$, this compound preferably being BCNU (N,N-bis(2-chloroethyl)-N-nitrosourea).

**14.** Composition according to the preceding claim, characterized in that it comprises at least one inhibitor of the transaminase involved in the conversion of 4-methylthio-2-oxobutanoic acid into methionine, such as the products mentioned in one of Claims 8 to 10.

**15.** Use of a composition defined according to any one of Claims 2 to 14 in a cosmetic composition or for the manufacture of a pharmaceutical composition, the composition being intended to induce apoptosis.

**16.** Process other than a therapeutic treatment method for preventing and/or combating light-induced or chronological ageing of the skin, characterized in that a cosmetic composition which induces apoptosis, according to one of Claims 2 to 14, is applied to the skin.

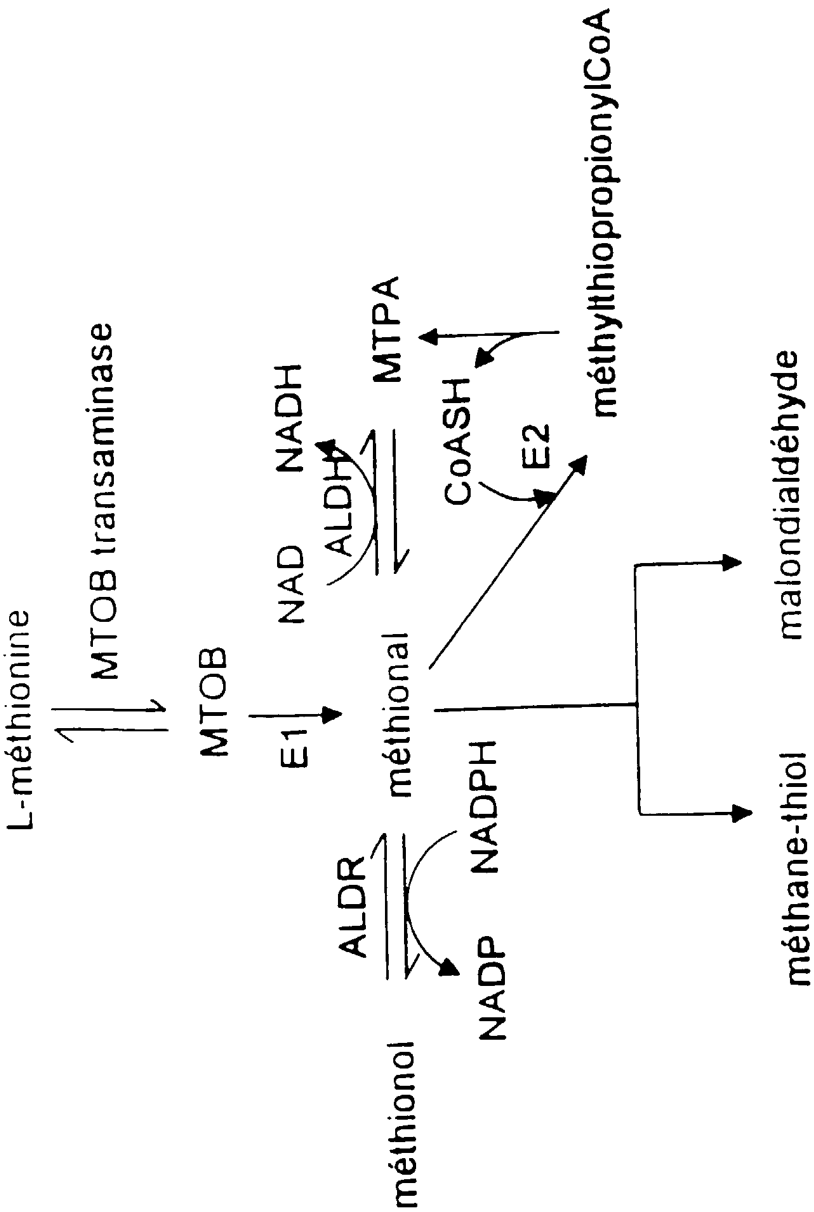
L-méthionine
MTOB transaminase
MTOB
E1
méthional
NAD
ALDH
NADH
MTPA
CoASH
E2
méthylthiopropionylCoA
ALDR
NADPH
NADP
méthionol
malondialdéhyde
méthane-thiol

FIGURE 1